# EUROPÄISCHE PATENTSCHRIFT

(11) **EP 2 241 253 B1**
(45) Veröffentlichungstag und Bekanntmachung des Hinweises auf die Patenterteilung: **15.06.2011**
(21) Anmeldenummer: 09157950.8
(22) Anmeldetag: 15.04.2009
(51) Int. Cl.: A61B 6/08, A61N 5/10, G06T 7/00

(54) **Verfahren zum Vervollständigen eines medizinischen Bilddatensatzes**
Method for completing a medicinal image database
Procédé destiné à compléter un ensemble de données d'images médicales

(43) Veröffentlichungstag der Anmeldung: 20.10.2010
(73) Patentinhaber: BrainLAB AG, 85622 Feldkirchen (DE)
(72) Erfinder: Berlinger, Kajetan, 80798, München (DE); Erbel, Stephan, 81825, München (DE)
(74) Vertreter: Schwabe - Sandmair - Marx

(56) Entgegenhaltungen:
- EP-A- 1 142 536
- WO-A-03/105069
- RUCHALA KENNETH J ET AL: "Methods for improving limited field-of-view radiotherapy reconstructions using imperfect a priori images" MEDICAL PHYSICS, AIP, MELVILLE, NY, US, Bd. 29, Nr. 11, 1. November 2002 (2002-11-01), Seiten 2590-2605, XP012011655 ISSN: 0094-2405

## Beschreibung

Die vorliegende Erfindung betrifft ein Verfahren und eine Vorrichtung zur Erzeugung eines vollständigen medizinischen Bilddatensatzes aus einem unvollständigen Bilddatensatz.

Im Bereich der Medizin ist es oftmals notwendig, über eine exakte Abbildung eines Körpers zu verfügen, um beispielsweise einen operativen Eingriff oder eine Bestrahlungsbehandlung zu planen beziehungsweise durchzuführen. Dabei ist es notwendig, die Abbildung möglichst unmittelbar vor der Bestrahlung beziehungsweise Behandlung zu gewinnen, da sich ein Körper auch innerhalb kurzer Zeiträume stark verändern kann. Derartige Abbildungen werden überwiegend mittels Kegelstrahl-Computertomographen (Cone-Beam Computed Tomograph, CBCT) gewonnen, da diese relativ einfache Geräte zur 3D-Bildgebung darstellen und zur Anfertigung der Abbildung nur wenig Zeit benötigen. Sie haben jedoch den Nachteil, aufgrund des Kegelstrahls nur einen beschränkten Erfassungsbereich aufzuweisen, der oftmals nicht den gesamten relevanten Bereich des Körpers umfasst. Darüber hinaus sind ihre räumliche Auflösung und ihr Dynamikumfang geringer als bei Fächerstrahl-Computertomographen (Fan-Beam Computed Tomograph, CT) oder Magnetresonanztomographen (MRT).

Zur Vermeidung dieses Nachteils schlägt das Patent US 6,915,005 B1 vor, zeitlich vor der CBCT-Abbildung eine CT-Abbildung anzufertigen, die einen größeren Bereich des Körpers erfasst als die CBCT-Abbildung, die CT-Abbildung auf die CBCT-Abbildung auszurichten, die ausgerichtete CT-Abbildung in ein Sinogramm umzuwandeln und Bereiche des Sinogramms der CBCT-Abbildung außerhalb des Erfassungsbereichs der CBCT-Abbildung aus dem Sinogramm der ausgerichteten CT-Abbildung zu ergänzen.

WO03/105069 offenbart ein Verfahren und Vorrichtung wobei für die Vervollständigung eines medizinischen Bilddatensatzes einen präoperativen vollständigen Bilddatensatz und einen intraoperativen unvollständigen Bilddatensatz benutzt werden.

Es ist die Aufgabe der vorliegenden Erfindung, das Verfahren und die Vorrichtung, wie sie aus dem Stand der Technik bekannt sind, dahingehend fortzubilden, dass die Erzeugung des vollständigen medizinischen Bilddatensatzes schneller möglich ist und der vervollständigte medizinische Bilddatensatz genauer mit dem aktuellen Zustand des Körpers übereinstimmt.

Diese Aufgabe wird gelöst durch das Verfahren gemäß Patentanspruch 1, das Computerprogramm gemäß Patentanspruch 11 und die Vorrichtung gemäß Patentanspruch 12. Vorteilhafte Ausgestaltungsformen sind den abhängigen Patentansprüchen zu entnehmen.

Gemäß dem erfindungsgemäßen Verfahren zur Erzeugung eines vollständigen medizinischen Bilddatensatzes aus einem unvollständigen Bilddatensatz wird ein erster Bilddatensatz bereitgestellt, der eine Abbildung eines ersten Bereichs eines Körpers inklusive mindestens eines Teils der Oberfläche des Körpers zu einem ersten Zeitpunkt repräsentiert. Der erste Bilddatensatz ist bevorzugt vollständig, aber zum Zeitpunkt der Behandlung unter Umständen nicht mehr aktuell. Weiterhin wird ein zweiter, unvollständiger Bilddatensatz bereitgestellt, der eine Abbildung eines zweiten Bereichs des Körpers zu einem zweiten Zeitpunkt repräsentiert, wobei sich der erste Bereich und der zweite Bereich überschneiden. Der zweite Zeitpunkt ist später als der erste Zeitpunkt. Ferner wird ein dritter Datensatz bereitgestellt, der die Kontur des Körpers in Form von Punkten auf der Oberfläche des Körpers im Wesentlichen zum zweiten Zeitpunkt repräsentiert.

Die Formulierung "im Wesentlichen zum zweiten Zeitpunkt" bedeutet, dass der Zeitpunkt, zu dem der dritte Datensatz gewonnen wird, gar nicht oder nur geringfügig vom zweiten Zeitpunkt abweicht. Der zeitliche Abstand zwischen der Erzeugung des dritten Datensatzes und dem zweiten Zeitpunkt ist bedeutend geringer, beträgt beispielsweise maximal ein zwanzigstel, bevorzugt maximal ein hundertstel, des zeitlichen Abstands zwischen dem ersten Zeitpunkt und dem zweiten Zeitpunkt. Der zweite Bilddatensatz und der dritte Datensatz werden bevorzugt gleichzeitig oder innerhalb weniger Minuten angefertigt, während die Zeitspanne zwischen dem ersten Zeitpunkt und dem zweiten Zeitpunkt mehrere Stunden, Tage oder gar Wochen betragen kann.

Gemäß dem vorliegenden Verfahren wird weiterhin der erste Bilddatensatz unter Berücksichtigung des dritten Datensatzes an den zweiten Bilddatensatz angepasst. Dies bedeutet, dass der erste Bilddatensatz möglichst gut mit dem zweiten Bilddatensatz in Übereinstimmung gebracht wird, beispielsweise durch Verschiebung und/oder Verdrehung des ersten Bilddatensatzes gegenüber dem zweiten Bilddatensatz und/oder einer beliebigen Verzerrung des ersten Bilddatensatzes. Verfahren zum Anpassen eines Bilddatensatzes an einen anderen Bilddatensatz sind aus dem Stand der Technik bekannt, beispielsweise unter den Bezeichnungen Image Matching oder Image fusion. Abschließend wird der angepasste erste Bilddatensatz als vollständiger medizinischer Bilddatensatz übernommen.

Werden hierin Daten, Regionen, Bereiche oder Bilder "bereitgestellt", so bedeutet dies, dass sie für die Nutzung durch das erfindungsgemäße Verfahren bereit sind. Diesen Zustand des "Bereitstehens" können die Daten, Regionen, Bereiche oder Bilder z.B. durch Erfassen der Daten, Regionen, Bereiche oder Bilder (z.B. durch Analysegeräte) oder durch Eingeben der Daten, Regionen, Bereiche oder Bilder (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen. Vor dem Bereitstellen, insbesondere vor dem Abspeichern, können die Daten, Regionen, Bereiche oder Bilder auch in einem Schritt des Verfahrens bestimmt, insbesondere berechnet werden.

Nach dem erfindungsgemäßen Verfahren wird der vollständige medizinische Bilddatensatz dadurch gewonnen, dass ein erster, bevorzugt vollständiger Bilddatensatz, der zu einem früheren Zeitpunkt erzeugt wurde, in möglichst gute Übereinstimmung mit einem zweiten, unvollständigen, aktuellen Bilddatensatz gebracht wird, wobei Informationen über die Oberfläche des Körpers in Form eines aktuellen dritten Datensatzes berücksichtigt werden. Durch die Berücksichtigung des dritten Datensatzes wird verhindert, dass der erste Bilddatensatz wesentlich oder überhaupt über die aktuelle Oberfläche des Körpers hinaus verschoben, verdreht oder verzerrt wird. Somit wird eine zusätzliche Information bei der Anpassung des ersten Bilddatensatzes berücksichtigt. Außerdem werden auch in dem Bereich, der von dem zweiten Bilddatensatz erfasst wird, die Informationen nicht aus dem zweiten Bilddatensatz, sondern aus dem ersten Bilddatensatz verwendet. Dies hat den Vorteil, dass der erste Bilddatensatz als CT- oder MRT-Bilddatensatz einen größeren Dynamikumfang aufweist als ein zweiter Bilddatensatz, der mittels eines CBCT erzeugt wurde. Der Dynamikumfang eines CBCT ist üblicherweise deutlich geringer als der eines CT oder MRT, der üblicherweise Werte zwischen -1000 und +3000 auf der Hounsfield-Skala generiert.

Weiterhin erfolgt die Erzeugung des vollständigen Bilddatensatzes nicht durch Transformation in ein Sinogramm, Ergänzung des Sinogramms und Rücktransformation in den Bildbereich, wie im Stand der Technik, sondern direkt im Bildbereich. Dadurch entfallen der Zeitaufwand und die Ungenauigkeiten, die bei der mehrfachen Transformation entstehen.

In einer Ausgestaltungsform der Erfindung erfolgt das Anpassen des ersten Bilddatensatzes an den zweiten Bilddatensatz mittels elastischer Bildfusion (elastic image fusion). Das Prinzip der elastischen Bildfusion ist dem Fachmann auf dem Gebiet der medizinischen Bildgebung bekannt. Es beruht auf einem iterativen Prozess, bei dem der erste Bilddatensatz schrittweise modifiziert und der modifizierte Bilddatensatz mit dem zweiten Bilddatensatz verglichen wird. Mögliche Modifikationen sind Verschiebungen, Drehungen oder Verzerrungen des Bilddatensatzes, die beliebig kombiniert werden können. Die Oberfläche des Körpers im modifizierten ersten Bilddatensatz wird auch als virtuelle Kontur bezeichnet. Der Vergleich des modifizierten ersten Bilddatensatzes und des zweiten Bilddatensatzes ergibt ein Ähnlichkeitsmaß, das die Ähnlichkeit der beiden Bilddatensätze repräsentiert. Diejenige Modifikation des ersten Bilddatensatzes, die zum größten Ähnlichkeitsmaß führt, führt zu einem ersten Bilddatensatz, der dem zweiten Bilddatensatz bestmöglich entspricht und somit den aktuellen Zustand des Körpers am besten repräsentiert.

Aus dem Stand der Technik sind verschiedenste Algorithmen bekannt, mit denen sich die elastische Bildfusion implementieren und optimieren lässt. Eine Möglichkeit besteht beispielsweise in der Interpolation unter Verwendung von Thin-Plate-Splines. Ein Thin-Plate-Spline interpoliert eine Oberfläche, die an vorgegebenen Fixpunkten unverändert bleiben soll. Diese Oberfläche repräsentiert eine dünne Metallplatte (thin metal plate), die bezogen auf die Verformungsenergie in die ökonomischste Form verformt wird, also die Verformungsenergie minimiert wird. Die Interpolation mittels Thin-Plate-Splines ist an sich wie in ihren Ableitungen stetig, hat keine freien Parameter, die manuell gesetzt werden müssen, und besitzt eine geschlossene Lösung.

Bei der elastischen Bildfusion wird der dritte Datensatz beispielsweise dadurch berücksichtigt, dass der Abstand der Punkte des dritten Datensatzes von korrespondierenden Punkten im ersten Datensatz bei der Bildfusion in das Ähnlichkeitsmaß eingeht. Dies bedeutet, dass sich das Ähnlichkeitsmaß nicht nur aus dem Bildvergleich des modifizierten ersten Bilddatensatzes mit dem zweiten Bilddatensatz ergibt, sondern zusätzlich aus dem Abstand der Oberfläche des Körpers, die vom dritten Datensatz repräsentiert wird, von derjenigen Oberfläche im modifizierten ersten Bilddatensatz. Dadurch wird verhindert, dass der modifizierte erste Bilddatensatz eine virtuelle Kontur des Körpers enthält, die deutlich von der tatsächlichen Kontur des Körpers abweicht. Dabei ist es möglich, das Überschreiten und das Unterschreiten der durch den dritten Datensatz repräsentierten Kontur des Körpers durch die virtuelle Kontur im modifizierten ersten Bilddatensatz unterschiedlich stark in das Ähnlichkeitsmaß einzubeziehen. So kann beispielsweise eine Überschreitung der tatsächlichen Kontur des Körpers das Ähnlichkeitsmaß stärker verringern als eine vergleichbare Unterschreitung der tatsächlichen Kontur.

Alternativ werden bei der Bildfusion keine Modifikationen des ersten Datensatzes zugelassen, bei denen der vom modifizierten ersten Datensatz repräsentierte erste Bereich über die vom dritten Datensatz repräsentierte Kontur des Körpers hinausgeht. Dies bedeutet, dass der dritte Datensatz eine harte Grenze für mögliche Modifikationen des ersten Bilddatensatzes darstellt.

In einer alternativen Ausgestaltungsform erfolgt die Anpassung des ersten Bilddatensatzes an den zweiten Bilddatensatz schrittweise. In einem ersten Schritt wird der erste Bilddatensatz ohne Berücksichtung des dritten Datensatzes an den zweiten Bilddatensatz angepasst. Dabei kann jedes Anpassungsverfahren verwendet werden, beispielsweise auch die elastische Bildfusion. In einem zweiten Schritt wird der im ersten Schritt angepasste erste Bilddatensatz segmentiert, wobei ein erstes Segment den Überschneidungsbereich des ersten Bereichs mit dem zweiten Bereich enthält und ein zweites Segment den Rest des ersten Bilddatensatzes enthält. Das bedeutet, dass das erste Segment den Bereich des Körpers repräsentiert, der im Erfassungsbereich beispielsweise des CBCT liegt. Alle Daten außerhalb dieses Bereichs sind im zweiten Segment enthalten.

In einem dritten Schritt wird dieses zweite Segment des ersten Bilddatensatzes unter Berücksichtigung des dritten Datensatzes angepasst. Auch für diese Anpassung können verschiedene Verfahren, beispielsweise elastische Bildfusion, verwendet werden. In diesem dritten Schritt wird der dritte Datensatz vorzugsweise dadurch berücksichtigt, dass er eine Begrenzung darstellt, in die das zweite Segment des ersten Bilddatensatzes eingepasst wird. Dabei wird das zweite Segment bevorzugt so angepasst, dass der Übergang zum ersten Segment stetig ist. Das bedeutet, dass das zweite Segment an der Übergangsfläche zum ersten Segment nicht verändert wird.

Durch die schrittweise Anpassung des ersten Bilddatensatzes an den zweiten Bilddatensatz werden die beiden Segmente des ersten Bilddatensatzes getrennt optimiert, wodurch im Überschneidungsbereich des ersten Bereichs mit dem zweiten Bereich die bestmögliche Übereinstimmung des ersten Bilddatensatzes mit dem zweiten Bilddatensatz erzielt wird, während das zweite Segment unter den vom dritten Datensatz gegebenen Randbedingungen modifiziert wird und somit bestmöglich die aktuelle Kontur des Körpers wiedergibt.

Bei der elastischen Bildfusion wird beispielsweise ein Verformungsfeld berechnet, das bevorzugt in einer dreidimensionalen Matrix für die Voxel des ersten Bilddatensatzes jeweils einen Verschiebungsvektor enthält. Dabei kann für jeden Voxel oder nur eine Teilmenge der Voxel ein Verschiebungsvektor vorgesehen sein. Bei der Berücksichtigung des dritten Datensatzes wird das Verformungsfeld an die Kontur im dritten Datensatz angepasst, beispielsweise anhand von korrespondierenden Kontur-Kontrollpunktpaaren bestehend aus einem Oberflächenpunkt im ersten Bilddatensatz und einem korrespondierenden Oberflächenpunkt im dritten Datensatz. Anschließend wird das Verformungsfeld auf den ersten Bilddatensatz angewendet. Dabei erfolgt eine Interpolation der Daten beispielsweise mittels Thin-Plate-Splines. Bei der Anwendung der vorliegenden Erfindung auf zweidimensionale Bilddaten ist die Matrix der Verschiebungsvektoren bevorzugt ebenfalls zweidimensional.

Wie bereits erläutert, wird der erste Bilddatensatz bevorzugt mittels Computertomographie (CT) oder Magnetresonanztomographie (MRT) ermittelt. Diese erzeugen Abbildungen mit hoher Auflösung und großen Dynamikumfang.

Der zweite Bilddatensatz wird bevorzugt mittels Kegelstrahl-Computertomographie (CBCT) ermittelt. Ein solcher Bilddatensatz verfügt über einen geringeren Dynamikumfang, lässt sich jedoch schneller und mit weniger Hardwareaufwand erzeugen als ein CT- oder MRT-Datensatz.

Der dritte Datensatz wird beispielsweise durch Laserabtastung der Oberfläche oder eines Teils der Oberfläche des Körpers gewonnen. Ein mögliches Verfahren zur Laserabtastung ist detailliert in der Europäischen Patentanmeldung EP 1 142 536 A1 der Anmelderin beschrieben. Dabei wird die zu erfassende Oberfläche des Körpers in den Erfassungsbereich eines durch mindestens zwei Kameras unterstützten Navigationssystems gebracht, welches computergestützt die dreidimensionalen Raumpositionen von Lichtmarkierungen erfasst. Mittels eines Lichtstrahls, bevorzugt eines eng fokussierten Laserstrahls, werden auf der Oberfläche des zu referenzierenden Körpers Lichtmarkierungen erzeugt, deren dreidimensionale Position von dem kameragestützten Navigationssystem bestimmt wird. Aus den Positionen und Ausrichtungen der Kameras sowie den von ihnen erzeugten Bildern wird die Position der Lichtmarkierung stereoskopisch berechnet. Optional wird aus der Größe der Lichtmarkierung im Bild einer Kamera eine zusätzliche Information über die Entfernung der Lichtmarkierung von der Kamera ermittelt. Die dreidimensionalen Positionen der abgetasteten Oberflächenpunkte werden zum dritten Datensatz zusammengefasst. Alternativ besteht die Möglichkeit, ein Gitter von Laserstrahlen auf die Oberfläche des Körpers zu projizieren, das Gitter mittels mindestens zwei Kameras zu erfassen und daraus Positionen von Oberflächenpunkten zu berechnen, die im dritten Datensatz abgelegt werden.

Alternativ oder zusätzlich wird der dritte Datensatz durch das Erfassen von Markierungen auf dem Körper gewonnen. Bei einer solchen Markierung kann es sich beispielsweise um einen Marker oder eine Markervorrichtung handeln.

Aufgabe eines Markers ist, von einer Markererfassungseinrichtung (z.B. einer Kamera oder einem Ultraschallempfänger) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Weiterhin alternativ oder zusätzlich wird der dritte Datensatz aus Röntgenbildern gewonnen, die auf dem Körper aufgebrachte Markierungen enthalten. Dazu können beispielsweise die Röntgenbilder genutzt werden, die ein Kegelstrahl-Computertomograph bei der Ermittlung des zweiten Bilddatensatzes erzeugt. Somit ist keine weitere Hardware zur Gewinnung des dritten Datensatzes notwendig. Die Markierungen, bevorzugt in Form von Metallplättchen oder Metallkugeln, werden auf dem Körper aufgebracht und sind in den einzelnen Röntgenbildern des CBCT sichtbar. Eine weitere Möglichkeit besteht darin, Markierungen, bevorzugt metallische Markierungen, in ein Kleidungsstück zu integrieren, das eng an dem Körper anliegt.

Weiterhin alternativ kann der dritte Datensatz durch Abtasten der Oberfläche des Körpers mittels eines Pointers gewonnen werden. Dabei wird die Spitze des Pointers auf verschiedene Punkte der Oberfläche des Körpers aufgesetzt und die Position der Spitze ermittelt.

Ein Pointer ist ein Stab mit einem oder mehreren, vorteilhaft zwei, daran befestigten Marker, wobei der Pointer dazu verwendet werden kann, einzelne Koordinaten, insbesondere Raumkoordinaten (also dreidimensionale Koordinaten), an einem Körper abzugreifen. Dabei führt ein Benutzer den Pointer (insbesondere einen Teil des Pointers, der eine definierte - vorteilhaft feste - Lage bezüglich des wenigstens einen an dem Pointer angebrachten Markers innehat) so an die den Koordinaten entsprechende Position, dass über eine Erfassung des Markers an dem Pointer durch ein chirurgisches Navigationssystem die Position des Pointers bestimmt werden kann. Insbesondere ist die relative Lage zwischen den Markern des Pointers und dem zum Abgreifen von Koordinaten verwendeten Teil des Pointers (insbesondere der Pointerspitze) bekannt. Das chirurgische Navigationssystem ermöglicht dann die Berechnung der Position (der dreidimensionalen Koordinaten) des den Körper berührenden Teils des Pointers und damit des berührten Punktes auf der Körperoberfläche, wobei die Berechnung automatisch und/oder durch Eingreifen des Benutzers erfolgen kann.

Die vorliegende Erfindung betrifft weiterhin ein Computerprogramm, das, wenn es auf einer Recheneinheit ausgeführt wird, einen oder mehrere der vorstehend geschilderten Verfahrensschritte ausgeführt.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speichermedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisungen ausführt, verkörpert sind bzw. ist. Ein solches System kann ein Computer sein, ein Computer kann eine Datenverarbeitungsvorrichtung mit Mitteln zum Ausführen der Computerprogrammelemente und/oder des erfindungsgemäßen Programms sein. Im Rahmen dieser Erfindung kann ein auf einem Computer verwendbares oder computerlesbares Medium irgendein Medium sein, das das Programm zur Verwendung auf oder in Verbindung mit dem System, Apparat oder der Einrichtung, das bzw. der bzw. die die Anweisungen ausführt, beinhalten, speichern, übermitteln, fortpflanzen oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise verarbeitet werden könnte. Das Computerprogrammprodukt und jegliche Software und/oder Hardware, das bzw. die hier beschrieben werden, bilden in den beispielhaften Ausführungsforrnen die verschiedenen Mittel zum Ausführen der Funktionen der Erfindung.

Das erfindungsgemäße Verfahren ist insbesondere ein Datenverarbeitungsverfahren. Das Datenverarbeitungsverfahren wird bevorzugt durch technische Mittel, insbesondere einen Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens, Anpassens oder Vergleichens von einem Computer ausgeführt. Schritte des Definierens von z. B. Bereichen oder Werten sind insbesondere Schritte des Festlegens von Daten im Rahmen des technischen Datenverarbeitungsverfahrens, insbesondere im Rahmen eines Programms. Schritte des Modifizierens stellen insbesondere eine Änderung der Daten mittels des Computers dar. Schritte des Ermittelns umfassen insbesondere die Abfrage von Werten, die an einer Schnittstelle des Computers anliegen und die durch technische Mittel, wie z. B. eine Abtasteinrichtung, erzeugt wurden. Diese Werte werden insbesondere von der Schnittstelle in Daten umgewandelt, die von dem Computer verarbeitet werden können.

Die vorliegende Erfindung umfasst weiterhin eine Vorrichtung zur Erzeugung eines vollständigen medizinischen Bilddatensatzes aus einem unvollständigen Bilddatensatz. Die Vorrichtung umfasst eine Schnittstelle zum Empfang eines ersten Bilddatensatzes, der eine Abbildung eines ersten Bereiches eines Körpers inklusive eines Teils der Oberfläche des Körpers zu einem ersten Zeitpunkt repräsentiert. Über die Schnittstelle kann die Vorrichtung beispielsweise mit einer medizinischen Bilderzeugungseinrichtung für einen Computertomographen oder einem Magnetresonanztomographen verbunden sein. Alternativ kann die Vorrichtung über die Schnittstelle mit einem Speicher verbunden sein, in dem der erste Bilddatensatz abgelegt ist. Dieser Speicher kann auch Bestandteil der Vorrichtung sein.

Die Vorrichtung umfasst weiterhin eine Einrichtung zur Erzeugung eines zweiten, unvollständigen Bilddatensatzes, der eine Abbildung eines zweiten Bereichs des Körpers zu einem zweiten Zeitpunkt repräsentiert, wobei sich der erste Bereich und der zweite Bereich überschneiden. Bei dieser Einrichtung handelt es sich bevorzugt um einen Kegelstrahl-Computertomographen.

Weiterhin umfasst die Vorrichtung eine Einrichtung zur Erzeugung eines dritten Datensatzes, der die Kontur des Körpers im Wesentlichen zum zweiten Zeitpunkt repräsentiert. Bei dieser Einrichtung handelt es sich beispielsweise um eine Laserabtasteinrichtung, wie sie vorstehend bereits beschrieben wurde.

Weiterhin umfasst die Vorrichtung eine Recheneinheit, die dazu eingerichtet ist, den ersten Bilddatensatz an den zweiten Bilddatensatz unter Berücksichtigung des dritten Datensatzes anzupassen und den angepassten ersten Bilddatensatz als vollständigen Bilddatensatz zu übernehmen.

Optional sind in der Vorrichtung weitere Komponenten vorgesehen oder vorhandene Komponenten wie die Recheneinheit so ausgebildet, dass sie geeignet sind, einen oder mehrere der vorgenannten Verfahrensschritte auszuführen.

In einer Ausgestaltungsform der Erfindung weist die Vorrichtung weiterhin eine Radiotherapie-Einrichtung auf, die beispielsweise einen Linac (Linear Accelerator) enthält und die auf Basis des vollständigen Bilddatensatzes ansteuerbar ist. Aus dem vollständigen Bilddatensatz lässt sich ein Behandlungsplan ableiten, anhand dessen die Radiotherapie-Einrichtung eingerichtet und aktiviert wird.

Der Therapie-Strahl, der von der Radiotherapie-Einrichtung erzeugt wird, kann optional zur Bildgebung, also insbesondere zur Erzeugung des zweiten Bilddatensatzes, verwendet werden. Der Therapie-Strahl weist üblicherweise einen höheren Energielevel auf als ein Röntgenstrahl, beispielsweise im Bereich von Megavolt (MV) im Vergleich zu Kilovolt (kV) bei Röntgenstrahlung. Bilddaten, die mittels des Therapie-Strahls gewonnen werden, werden daher auch als MV CBCT bezeichnet. Die MV CBCT-Projektionsbilder können alleine oder in Kombination mit den kV CBCT-Projektionsbildern einer Röntgeneinrichtung zur Rekonstruktion von dreidimensionalen Bilddaten genutzt werden.

Bevorzugt ist zumindest die Einrichtung zur Erzeugung des zweiten Bilddatensatzes an einem Träger, der auch als Gantry bezeichnet wird, angeordnet, wobei der Träger rotatorisch und/oder translatorisch beispielsweise gegenüber einem Tisch, auf dem sich der Körper befindet, beweglich ist. Optional sind weitere Einrichtungen, wie die Einrichtung zur Erzeugung eines dritten Datensatzes oder die Radiotherapie-Einrichtung, oder Komponenten der Einrichtungen an dem gleichen Träger angeordnet. Dadurch ist die relative Lage der Einrichtungen bzw. Komponenten zueinander bekannt und die Lage der Einrichtungen bzw. Komponenten gegenüber dem Körper ist durch Bewegung eines einzigen Trägers veränderbar.

Die vorliegende Erfindung soll anhand eines Ausführungsbeispiels näher erläutert werden. Dabei zeigt:
- Figur 1: die Kontur eines Körpers, den Erfassungsbereich eines CBCT und einige Oberflächenpunkte und
- Figur 2: eine erfindungsgemäße Vorrichtung.

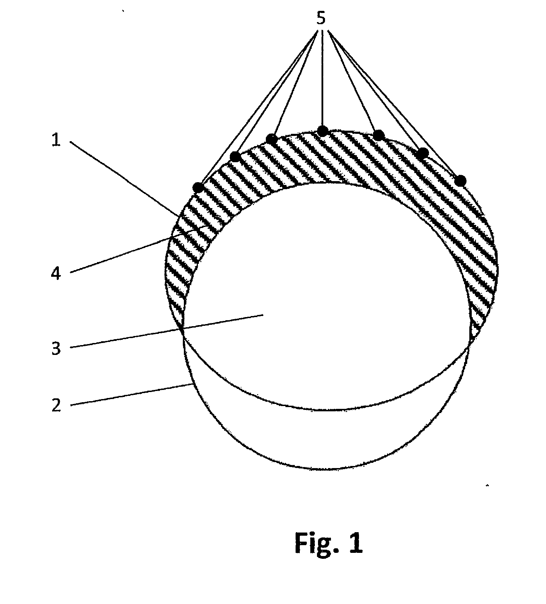

Figur 1 zeigt schematisch einen Querschnitt durch einen Körper 1, dessen Oberfläche im vorliegenden Ausführungsbeispiel eine elliptische Form aufweist. Durch Kreise symbolisiert sind einige Punkte 5 auf der Oberfläche des Körpers 1. Die Figur 1 zeigt weiterhin einen Erfassungsbereich 2 eines Kegelstrahl-Computertomographen. Der Körper 1 und der Erfassungsbereich 2 überschneiden sich in einem Überschneidungsbereich 3. Der schraffiert dargestellte Bereich 4 des Körpers 1 wird nicht vom Erfassungsbereich 2 abgedeckt.

Die Figur 1 zeigt den Körper 1 und die Bereiche 2, 3 und 4 aus Darstellungsgründen zweidimensional. In der Praxis werden sie jedoch überwiegend dreidimensional sein. Der Erfassungsbereich 2 ist beispielsweise kugelförmig.

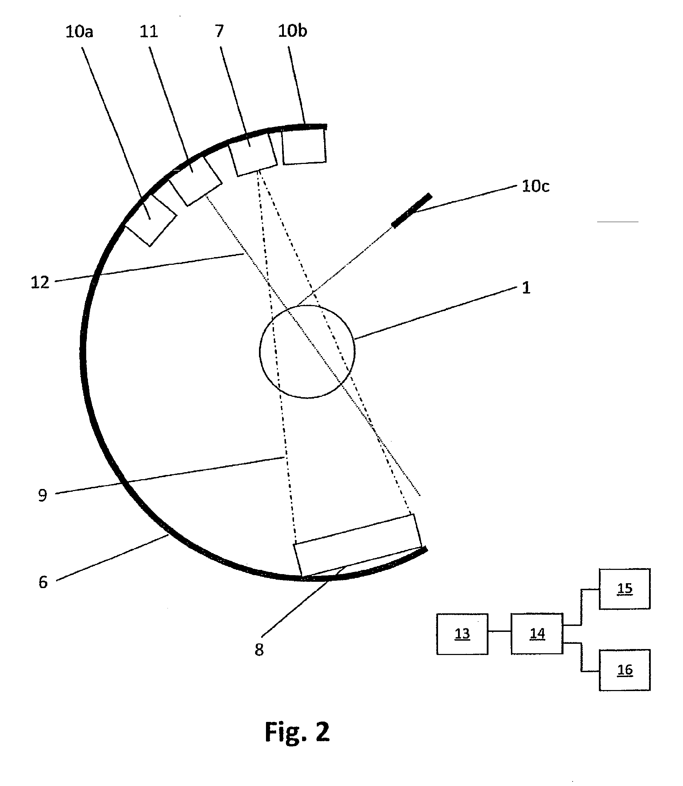

Die Figur 2 zeigt eine erfindungsgemäße Vorrichtung, die beispielsweise auf einem C-förmigen oder kreisbogenförmigen Träger 6 basiert. Als Träger der einzelnen Komponenten der Vorrichtung kann jedoch jede andere geeignete Konstruktion vorgesehen werden, beispielsweise eine Röntgeneinrichtung in Form eines sogenannten C-Bogens, die um zusätzliche Einrichtungen erweitert wird.

Am Träger 6 sind eine Röntgenquelle 7 und ein Röntgendetektor 8 angeordnet, sodass der kegelförmige Röntgenstrahl 9 der Röntgenquelle 7 den Körper 1 durchstrahlt und auf den Röntgendetektor 8 trifft. Durch eine Drehung des Trägers 6 um seine Drehachse, die im vorliegenden Beispiel senkrecht auf der Zeichenebene steht, lassen sich Röntgenaufnahmen des Körpers 1 aus verschiedenen Blickrichtungen erzeugen und zu einer dreidimensionalen Abbildung verrechnen.

Am Träger 6 sind weiterhin die beiden Kameras 10a und 10b angeordnet. Diese Kameras können anstatt am Träger 6 auch ortsfest bezüglich des Körpers 1 angeordnet sein, beispielsweise auf einem Stativ oder einer Decke oder Wand eines Raumes. Mittels eines Lasergenerators 10c wird ein Laserstrahl erzeugt, der auf den Körper 1 gerichtet werden kann. Die Kameras 10a und 10b erfassen den vom Lasergenerator 10c erzeugten Lichtfleck auf der Oberfläche des Körpers 1. Auf den Bildern der Kameras 10a und 10b lässt sich die Position des Lichtflecks im Raum und damit die Position des angeleuchteten Punkts auf der Oberfläche des Körpers 1 bestimmen.

Weiterhin am Träger 6 angeordnet ist ein optionaler Strahlerzeuger 11, der auch als Linear Accelerator (Linac) bezeichnet wird. Er dient dazu, einen Behandlungsstrahl 12 zu erzeugen, der beispielsweise zur Bestrahlung eines Tumors im Körper 1 geeignet ist. Der Strahlerzeuger 11 ist Bestandteil einer Radiotherapie-Einrichtung.

Zur korrekten Planung des Einsatzes der Radiotherapie-Einrichtung, aber auch für eine Vielzahl anderer medizinischer Anwendungen ist es notwendig, über eine Abbildung des Körpers 1 unmittelbar vor der Bestrahlung bzw. Behandlung zu verfügen. Eine solche Abbildung lässt sich mittels eines Kegelstrahl-Computertomographen, der die Röntgenquelle 7 und den Detektor 8 enthält, erzeugen. Wie aus Figur 1 ersichtlich ist, umfasst der Erfassungsbereich 2 des CBCT jedoch oftmals nicht den gesamten Körper 1. Es ist daher notwendig, den vom CBCT erzeugten unvollständigen Bilddatensatz zu einem vollständigen medizinischen Bilddatensatz zu ergänzen.

Dazu wird zunächst ein erster Bilddatensatz bereitgestellt, der eine Abbildung eines ersten Bereichs eines Körpers 1 inklusive zumindest eines Teils der Oberfläche des Körpers zu einem ersten Zeitpunkt repräsentiert. Im vorliegenden Beispiel umfasst der erste Bereich, im Querschnitt betrachtet, den kompletten Körper 1. Weiterhin wird ein zweiter, unvollständiger Bilddatensatz bereitgestellt, der eine Abbildung eines zweiten Bereichs 2 des Körpers 1 zu einem zweiten Zeitpunkt repräsentiert, wobei sich der erste Bereich und der zweite Bereich 2 überschneiden. Außerdem wird ein dritter Datensatz bereitgestellt, der die Kontur des Körpers 1 in Form von Punkten 5 auf der Oberfläche des Körpers 1 im Wesentlichen zum zweiten Zeitpunkt repräsentiert. Der zweite Zeitpunkt liegt beispielsweise unmittelbar vor der Behandlung bzw. Bestrahlung des Körpers 1. Der zweite Bilddatensatz wird, wie vorstehend beschrieben, mittels der Röntgenquelle 7 und dem Röntgendetektor 8 eines Kegelstrahl-Computertomographen ermittelt. Der dritte Datensatz wird dadurch ermittelt, dass Punkte 5 auf der Oberfläche des Körpers 1 mittels des Laserstrahls der Laserquelle 10c angestrahlt werden und die Reflektion des Laserstrahls auf der Oberfläche des Körpers 1 von den Kameras 10a und 10b erfasst wird. Aus den Bildern, die die Kameras 10a und 10b erzeugen, sowie der räumlichen Anordnung der Kameras zueinander lässt sich die Position des Reflektionspunkts und damit des Oberflächenpunkts 5 berechnen. Der zweite Bilddatensatz und der dritte Datensatz beziehen sich bevorzugt auf ein gemeinsames Koordinatensystem, das beispielsweise im Bezug auf den Körper 1 oder den Träger 6 definiert ist.

Der erste Bilddatensatz wird mittels eines Fächerstrahl-Computertomographen (CT) oder eines Magnetresonanztomographen (MRT) erzeugt. Der erste Zeitpunkt, zu dem der erste Bilddatensatz erzeugt wird, kann Minuten oder Stunden, aber auch Tage oder gar Wochen vor dem zweiten Zeitpunkt liegen.

Der dritte Datensatz wird im Wesentlichen zu dem Zeitpunkt erzeugt, zu dem der zweite Bilddatensatz erzeugt wird, also zum zweiten Zeitpunkt. Dies bedeutet, dass die Zeitpunkte der Erzeugung des dritten Datensatzes und des zweiten Bilddatensatzes näher zusammen liegen als der erste Zeitpunkt und der zweite Zeitpunkt. Bevorzugt werden der zweite Bilddatensatz und der dritte Datensatz gleichzeitig oder zumindest unmittelbar aufeinander folgend aufgenommen.

Im weiteren Verlauf des Verfahrens wird der erste Bilddatensatz unter Berücksichtung des dritten Datensatzes an den zweiten Bilddatensatz angepasst. Dies bedeutet, dass der erste Bilddatensatz derart modifiziert wird, dass er bestmöglich dem zweiten Bilddatensatz entspricht, wobei der dritte Datensatz eine Randbedingung für die Modifikation definiert. Der angepasste erste Bilddatensatz wird dann als vollständiger Bilddatensatz übernommen. Dies bedeutet, dass der zweite Bilddatensatz nicht in den vollständigen Bilddatensatz übernommen wird, sondern nur als Referenz zur Anpassung des ersten Bilddatensatzes dient, sodass der erste Bilddatensatz bestmöglich den Zustand des Körpers 1 zum zweiten Zeitpunkt repräsentiert.

Im vorliegenden Ausführungsbeispiel erfolgt das Anpassen des ersten Bilddatensatzes mittels elastischer Bildfusion. Dabei wird iterativ eine Vielzahl von modifizierten Bilddatensätzen erzeugt, indem der erste Bilddatensatz verschoben, verdreht oder verzerrt wird, wobei auch eine beliebige Kombination dieser Modifikationen möglich ist. Jeder modifizierte Bilddatensatz wird mit dem zweiten Bilddatensatz verglichen, was für jeden Bildvergleich zu einem Ähnlichkeitsmaß führt, das die Ähnlichkeit der beiden Bilddatensätze repräsentiert.

Im Folgenden werden zwei Varianten der Anpassung beschrieben. In der ersten Variante geht in das Ähnlichkeitsmaß der elastischen Bildfusion neben der Ähnlichkeit des modifizierten ersten Bilddatensatzes und des zweiten Datensatzes ein Abstandswert ein. Dieser Abstandswert bestimmt sich aus dem Abstand der Punkte 5, die vom dritten Datensatz repräsentiert werden, von der Oberfläche des Körpers 1 im modifizierten ersten Bilddatensatz. Bei der Modifikation des ersten Bilddatensatzes ergibt sich ein neuer, virtueller Verlauf der Oberfläche des Körpers 1 im modifizierten ersten Bilddatensatz. Diese modifizierte erste Oberfläche (oder virtuelle Kontur) wird mit der Kontur des Körpers, wie sie anhand der Punkte 5 im dritten Datensatz hinterlegt ist, verglichen. Je größer der Abstand der modifizierten Oberfläche im modifizierten ersten Bilddatensatz von der Oberfläche im dritten Datensatz ist, desto stärker wird das Ähnlichkeitsmaß, das sich aus dem Vergleich des modifizierten ersten Bilddatensatzes mit dem zweiten Bilddatensatz ergibt, verringert. Dies kann soweit gehen, dass das Ähnlichkeitsmaß auf Null reduziert wird, wenn einige oder alle Punkte 5 im modifizierten ersten Bilddatensatz innerhalb des Körpers 1 liegen, sich die virtuelle Kontur des Körpers 1 im modifizierten ersten Bilddatensatz also über die gemessene und vom dritten Datensatz repräsentierte Oberfläche des Körpers 1 zum zweiten Zeitpunkt hinaus erstreckt. In diesem Fall dient der dritte Datensatz als harte Grenze für mögliche Modifikationen des ersten Bilddatensatzes während der elastischen Bildfusion.

Der Abstandswert ist beispielsweise die Summe Abstände der einzelnen Punkte 5 von der virtuellen Kontur im modifizierten ersten Bilddatensatz oder deren Mittelwert. Der Abstand ist beispielsweise der minimale Abstand eines Punktes 5 von der virtuellen Kontur oder der Abstand zu einem korrespondierenden Punkt, beispielsweise einer Landmarke. Bei einer Landmarke handelt es sich um einen definierten, charakteristischen Punkt einer anatomischen Struktur, der bei der gleichen anatomischen Struktur mehrerer Patienten stets identisch oder mit hoher Ähnlichkeit wiederkehrt. Typische Landmarken sind beispielsweise die Epikondylen eines Oberschenkelknochens, die Spitzen der Querfortsätze bzw. des Domfortsatzes eines Wirbels oder Punkte wie die Nasenspitze oder das Ende der Nasenwurzel.

Gemäß einer zweiten Variante erfolgt das Anpassen des ersten Bilddatensatzes an den zweiten Bilddatensatz schrittweise. In einem ersten Schritt wird der erste Bilddatensatz ohne Berücksichtigung des dritten Datensatzes an den zweiten Bilddatensatz angepasst, beispielsweise mittels elastischer Bildfusion. Dies hat zur Folge, dass der angepasste erste Bilddatensatz und der zweite Bilddatensatz nach dem ersten Schritt im Überschneidungsbereich 3 optimal übereinstimmen. Dann wird der im ersten Schritt angepasste erste Bilddatensatz derart segmentiert, dass das erste Segment den Überschneidungsbereich 3 repräsentiert und ein zweites Segment den Rest des angepassten ersten Bilddatensatzes enthält. In einem dritten Schritt der Anpassung wird das zweite Segment des ersten Bilddatensatzes im zu ergänzenden Bereich 4 angepasst. Das zweite Segment des ersten Bilddatensatzes wird beispielsweise so angepasst, dass es sich optimal in den Bereich 4 einpasst, der einerseits von der Grenze des ersten Segments des modifizierten ersten Bilddatensatzes und andererseits von den Punkten 5 im dritten Datensatz begrenzt wird. Bei diesem Schritt kann als Nebenbedingung berücksichtigt werden, dass der Übergang vom ersten Segment zum zweiten Segment des ersten Bilddatensatzes stetig verlaufen soll, also die unmittelbar an das erste Segment angrenzenden Daten des zweiten Segments nicht oder nur geringfügig verändert werden.

Der Vorteil der zweiten Variante liegt darin, dass der erste Bilddatensatz im Erfassungsbereich 2 des CBCT optimal mit dem zweiten Bilddatensatz in Übereinstimmung gebracht wird, während gleichzeitig der erste Bilddatensatz optimal in die vom dritten Datensatz repräsentierte Kontur des Körpers 1 eingepasst wird.

Die Vorrichtung weist weiterhin eine Recheneinheit 13 auf, die mit dem Röntgengenerator 7 und dem Röntgendetektor 8 verbunden ist. Die Recheneinheit 13 aktiviert den Röntgengenerator 7 und empfängt die Ausgangssignale des Röntgendetektors 8. Die Recheneinheit 13 ist weiter dazu eingerichtet, den Träger 6 in verschiedene Positionen bezogen auf den Körper 1 zu bewegen. Aus den Ausgangssignalen des Röntgendetektors 8 in den verschiedenen Positionen des Trägers 6 berechnet die Recheneinheit 13 eine dreidimensionale Abbildung des Körpers 1, die in einem zweiten Bilddatensatz abgelegt wird. Die Recheneinheit 13 ist über eine Schnittstelle 14 mit einem Computertomographen 15 oder einem Speicher 16 verbunden. Der erste Bilddatensatz wird beispielsweise vom Computertomographen 15 erzeugt und der Recheneinheit 13 über die Schnittstelle 14 bereitgestellt. Alternativ ist der erste Bilddatensatz im Speicher 16 gespeichert und wird der Recheneinheit 13 über die Schnittstelle 14 bereitgestellt. Die Verbindungen der Recheneinheit 13 zu den am Träger 6 angeordneten Komponenten sind aus Gründen der Übersichtlichkeit in der Figur 2 nicht dargestellt.

Optional ist die Recheneinheit 13 auch mit den Kameras 10a und 10b verbunden, sodass sie aus den Ausgangssignalen der Kameras 10a und 10b sowie deren Lage die Position der Punkte 5 berechnen kann. Alternativ ist die Recheneinheit 13 über die Schnittstelle 14 oder eine nicht dargestellte Schnittstelle mit einer zweiten Recheneinheit verbunden, die wiederum mit den Kameras 10a und 10b verbunden ist und aus deren Ausgangsignalen sowie deren Lage die Position der Punkte 5 berechnet und der Recheneinheit 13 bereitstellt.

## Patentansprüche

1. Verfahren zur Erzeugung eines vollständigen medizinischen Bilddatensatzes aus einem unvollständigen Bilddatensatz, aufweisend die Verfahrensschritte
- Bereitstellen eines ersten Bilddatensatzes, der eine Abbildung eines ersten Bereiches eines Körpers (1) inklusive zumindest eines Teils der Oberfläche des Körpers (1) zu einem ersten Zeitpunkt repräsentiert;
- Bereitstellen eines zweiten, unvollständigen Bilddatensatzes, der eine Abbildung eines zweiten Bereichs (2) des Körpers (1) zu einem zweiten Zeitpunkt repräsentiert, wobei sich der erste Bereich und der zweite Bereich (2) überschneiden;
- Bereitstellen eines dritten Datensatzes, der die Kontur des Körpers (1) in Form von Punkten (5) auf der Oberfläche des Körpers (1) im Wesentlichen zum zweiten Zeitpunkt repräsentiert;
- Anpassen des ersten Bilddatensatzes an den zweiten Bilddatensatz unter Berücksichtigung des dritten Datensatzes; und
- Übernehmen des angepassten ersten Bilddatensatzes als vollständigen Bilddatensatz.

2. Verfahren nach Anspruch 1, wobei das Anpassen mittels elastischer Bildfusion erfolgt.

3. Verfahren nach Anspruch 2, der dritte Datensatz berücksichtigt wird, indem der Abstand der Punkte (5) des dritten Datensatzes von korrespondierenden Punkten im ersten Datensatz in das Ähnlichkeitsmaß bei der Bildfusion eingeht.

4. Verfahren nach Anspruch 3, bei der Bildfusion keine Modifikationen des ersten Bildatensatzes zugelassen werden, bei denen der vom ersten Datensatz repräsentierte erste Bereich über die vom dritten Datensatz repräsentierte Kontur des Körpers (1) hinausgeht.

5. Verfahren nach Anspruch 1 oder 2, wobei die Anpassung schrittweise erfolgt, wobei
- in einem ersten Schritt der erste Bilddatensatz ohne Berücksichtigung des dritten Datensatzes an den zweiten Bilddatensatz angepasst wird
- in einem zweiten Schritt der im ersten Schritt angepasste erste Bilddatensatz segmentiert wird, wobei ein erstes Segment den Überschneidungsbereich (3) des ersten Bereichs mit dem zweiten Bereich (2) enthält und ein zweites Segment den Rest des ersten Bilddatensatzes enthält, und
- in einem dritten Schritt das zweite Segment des ersten Bilddatensatzes unter Berücksichtigung des dritten Datensatzes angepasst wird.

6. Verfahren nach einem der Ansprüche 1 bis 5, wobei der erste Bilddatensatz mittels Computertomographie (CT) oder Magnetresonanztomographie (MRT) ermittelt wird.

7. Verfahren nach einem der Ansprüche 1 bis 6, wobei der zweite Bilddatensatz mittels Kegelstrahl-Computertomographie (CBCT) ermittelt wird.

8. Verfahren nach einem der Ansprüche 1 bis 7, wobei der dritte Datensatz durch Laserabtastung des Körpers (1) gewonnen wird.

9. Verfahren nach einem der Ansprüche 1 bis 8, wobei der dritte Datensatz durch das Erfassen von Markierungen auf dem Körper (1) gewonnen wird.

10. Verfahren nach einem der Ansprüche 1 bis 9, wobei der dritte Datensatz aus Röntgenbildern gewonnen wird, die auf den Körper aufgebrachte Markierungen enthalten.

11. Computerprogramm, das, wenn es auf einer Recheneinheit ausgeführt wird, die Verfahrensschritte nach einem der Ansprüche 1 bis 10 ausführt.

12. Vorrichtung zur Erzeugung eines vollständigen medizinischen Bilddatensatzes aus einem unvollständigen Bilddatensatz, aufweisend
- eine Schnittstelle (14) zum Empfang eines ersten Bilddatensatzes, der eine Abbildung eines ersten Bereiches eines Körpers (1) inklusive zumindest eines Teils der Oberfläche des Körpers (1) zu einem ersten Zeitpunkt repräsentiert; und
- eine Einrichtung (7, 8) zur Erzeugung eines zweiten, unvollständigen Bilddatensatzes, der eine Abbildung eines zweiten Bereichs (2) des Körpers (1) zu einem zweiten Zeitpunkt repräsentiert, wobei sich der erste Bereich und der zweite Bereich (2) überschneiden;
**gekennzeichnet durch**
- eine Einrichtung (10a, 10b, 10c) zur Erzeugung eines dritten Datensatzes, der die Kontur des Körpers (1) in Form von Punkten (5) auf der Oberfläche des Körpers (1) im Wesentlichen zum zweiten Zeitpunkt repräsentiert;
- eine Recheneinheit (13), die dazu eingerichtet ist, den ersten Bilddatensatz an den zweiten Bilddatensatz unter Berücksichtigung des dritten Datensatzes anzupassen und den angepassten ersten Bilddatensatz als vollständigen Bilddatensatz zu übernehmen.

13. Vorrichtung nach Anspruch 12, **gekennzeichnet durch** die Einrichtung zur Erzeugung des zweiten Bilddatensatzes ein Kegelstrahl-Computertomograph ist.

14. Vorrichtung nach einem der Ansprüche 12 oder 13, **gekennzeichnet durch** die Einrichtung zur Erzeugung des dritten Datensatzes eine Laserabtasteinrichtung ist.

15. Vorrichtung nach einem der Ansprüche 12 bis 14, die danüber hinaus eine Radiotherapie-Einrichtung (11) enthält, die auf Basis des vollständigen Bilddatensatzes angesteuert wird.

## Claims

1. A method for generating a complete medical image data set from an incomplete image data set, comprising the method steps of:
- providing a first image data set which represents an image of a first region of a body (1), including at least a part of the surface of the body (1), at a first point in time;
- providing a second, incomplete image data set which represents an image of a second region (2) of the body (1) at a second point in time, wherein the first region and the second region (2) overlap;
- providing a third data set which represents the contour of the body (1) in the form of points (5) on the surface of the body (1), substantially at the second point in time;
- adapting the first image data set to the second image data set by taking into account the third data set; and
- accepting the adapted first image data set as a complete image data set.

2. The method according to claim 1, wherein the first image data set is adapted by means of elastic image fusion.

3. The method according to claim 2, wherein the third data set is taken into account by incorporating the distance between the points (5) of the third data set and corresponding points in the first data set into the degree of similarity during image fusion.

4. The method according to claim 3, wherein no modifications of the first image data set in which the first region represented by the first data set exceeds the contour of the body (1) represented by the third data set are permitted during image fusion.

5. The method according to claim 1 or 2, wherein the first image data set is adapted in steps, wherein:
- in a first step, the first image data set is adapted to the second image data set without taking into account the third data set;
- in a second step, the first image data set which was adapted in the first step is segmented, wherein a first segment contains the overlap region (3) between the first region and the second region (2), and a second segment contains the rest of the first image data set; and
- in a third step, the second segment of the first image data set is adapted by taking into account the third data set.

6. The method according to any one of claims 1 to 5, wherein the first image data set is ascertained by means of computed tomography (CT) or magnetic resonance tomography (MRT).

7. The method according to any one of claims 1 to 6, wherein the second image data set is ascertained by means of cone beam computed tomography (CBCT).

8. The method according to any one of claims 1 to 7, wherein the third data set is obtained by laser-scanning the body (1).

9. The method according to any one of claims 1 to 8, wherein the third data set is obtained by detecting markings on the body (1).

10. The method according to any one of claims 1 to 9, wherein the third data set is obtained from x-ray images which contain markings attached to the body.

11. A computer program which, when it is run on a computational unit, performs the method steps according to any one of claims 1 to 10.

12. A device for generating a complete medical image data set from an incomplete image data set, comprising:
- an interface (14) for receiving a first image data set which represents an image of a first region of a body (1), including at least a part of the surface of the body (1), at a first point in time;
- a device (7, 8) for generating a second, incomplete image data set which represents an image of a second region (2) of the body (1) at a second point in time, wherein the first region and the second region (2) overlap;
**characterised by**:
- a device (10a, 10b, 10c) for generating a third data set which represents the contour of the body (1) in the form of points (5) on the surface of the body (1), substantially at the second point in time;
- a computational unit (13) which is configured to adapt the first image data set to the second image data set by taking into account the third data set, and to accept the adapted first image data set as a complete image data set.

13. The device according to claim 12, wherein the device for generating the second image data set is a cone beam computed tomograph.

14. The device according to any one of claims 12 or 13, wherein the device for generating the third data set is a laser scanning device.

15. The device according to any one of claims 12 to 14, further including a radiotherapy device (11) which is controlled on the basis of the complete image data set.

## Revendications

1. Procédé pour générer un ensemble complet de données d'image médicale à partir d'un ensemble incomplet de données d'image, comportant les étapes de procédé consistant à :
- fournir un premier ensemble de données d'image qui représente une image d'une première région d'un corps (1), incluant au moins une partie de la surface du corps (1), à un premier instant,
- fournir un deuxième ensemble incomplet de données d'image qui représente une image d'une seconde région (2) du corps (1) à un second instant, dans lequel la première région et la seconde région (2) se chevauchent,
- fournir un troisième ensemble de données qui représente le contour du corps (1) sous forme de points (5) sur la surface du corps (1) sensiblement au second instant,
- adapter le premier ensemble de données d'image au deuxième ensemble de données d'image en tenant compte du troisième ensemble de données, et
- accepter le premier ensemble de données d'image adapté comme un ensemble de données d'image complet.

2. Procédé selon la revendication 1, dans lequel l'adaptation s'effectue au moyen d'une fusion d'image élastique.

3. Procédé selon la revendication 2, dans lequel le troisième ensemble de données est pris en compte en incorporant la distance entre les points (5) du troisième ensemble de données et des points correspondants dans le premier ensemble de données dans le degré de similitude lors de la fusion d'image.

4. Procédé selon la revendication 3, dans lequel aucune modification du premier ensemble de données d'image n'est autorisée lors de la fusion d'image, dans lequel la première région représentée par le premier ensemble de données dépasse le contour du corps (1) représenté par le troisième ensemble de données.

5. Procédé selon la revendication 1 ou 2, dans lequel l'adaptation s'effectue par étapes, dans lequel
- lors d'une première étape, le premier ensemble de données d'image est adapté au deuxième ensemble de données d'image sans tenir compte du troisième ensemble de données,
- lors d'une deuxième étape, le premier ensemble de données d'image qui a été adapté à la première étape est segmenté, dans lequel un premier segment contient la région de chevauchement (3) entre la première région et la seconde région (2), et un second segment contient le reste du premier ensemble de données d'image, et
- lors d'une troisième étape, le second segment du premier ensemble de données d'image est adapté en tenant compte du troisième ensemble de données.

6. Procédé selon l'une quelconque des revendications 1 à 5, dans lequel le premier ensemble de données d'image est déterminé au moyen d'une tomographie par ordinateur (CT) ou d'une tomographie à résonance magnétique (MRT).

7. Procédé selon l'une quelconque des revendications 1 à 6, dans lequel le deuxième ensemble de données d'image est déterminé au moyen d'une tomographie par ordinateur à faisceau conique (CBCT).

8. Procédé selon l'une quelconque des revendications 1 à 7, dans lequel le troisième ensemble de données est obtenu par balayage laser du corps (1).

9. Procédé selon l'une quelconque des revendications 1 à 8, dans lequel le troisième ensemble de données est obtenu par détection de marquages sur le corps (1).

10. Procédé selon l'une quelconque des revendications 1 à 9, dans lequel le troisième ensemble de données est obtenu à partir d'images de rayons X qui contiennent des marquages appliqués sur le corps.

11. Programme informatique qui, lorsqu'il est exécuté sur une unité de calcul, met en oeuvre les étapes de procédé selon l'une quelconque des revendications 1 à 10.

12. Dispositif pour générer un ensemble complet de données d'image médicale à partir d'un ensemble incomplet de données d'image médicale, comportant :
- une interface (14) pour recevoir un premier ensemble de données d'image qui représente une image d'une première région d'un corps (1), incluant au moins une partie de la surface du corps (1), à un premier instant, et
- un dispositif (7, 8) pour générer un deuxième ensemble incomplet de données d'image qui représente une image d'une seconde région (2) du corps (1) à un second instant, dans lequel la première région et la seconde région (2) se chevauchent,
**caractérisé par**
- un dispositif (10a, 10b, 10c) pour générer un troisième ensemble de données qui représente le contour du corps (1) sous forme de points (5) sur la surface du corps (1) sensiblement au second instant,
- une unité de calcul (13) qui est configurée pour adapter le premier ensemble de données d'image au deuxième ensemble de données d'image en tenant compte du troisième ensemble de données et pour accepter le premier ensemble de données d'image adapté comme un ensemble de données d'image complet.

13. Dispositif selon la revendication 12, **caractérisé en ce que** le dispositif pour générer le deuxième ensemble de données d'image est un tomographe par ordinateur à faisceau conique.

14. Dispositif selon l'une quelconque des revendications 12 ou 13, **caractérisé en ce que** le dispositif pour générer le troisième ensemble de données est un dispositif de balayage laser.

15. Dispositif selon l'une quelconque des revendications 12 à 14, lequel contient en outre un dispositif de radiothérapie (11) qui est commandé sur la base de l'ensemble de données d'image complet.
